(19) Europäisches Patentamt
European Patent Office
Office européen des brevets

(11) **EP 1 733 755 B1**

(12) **EUROPEAN PATENT SPECIFICATION**

(45) Date of publication and mention
of the grant of the patent:
**19.11.2008 Bulletin 2008/47**

(51) Int Cl.:
*A61F 7/10* (2006.01)    *A61K 9/00* (2006.01)

(21) Application number: **06090077.6**

(22) Date of filing: **16.05.2006**

(54) **An apparatus containing a composition**

Vorrichtung zum Auftragen einer Zusammensetzung

Appareil pour l'application d'une composition

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.06.2005 EP 05105216**
**25.11.2005 EP 05111304**

(43) Date of publication of application:
**20.12.2006 Bulletin 2006/51**

(73) Proprietor: **RIZK, Nelly Kamel**
**South Croydon,**
**Surrey CR2 6DP (GB)**

(72) Inventor: **RIZK, Nelly Kamel**
**South Croydon,**
**Surrey CR2 6DP (GB)**

(56) References cited:
**EP-A- 0 404 334**        **EP-A- 1 195 173**
**WO-A-95/04522**        **DE-A1- 10 252 917**
**GB-A- 2 011 548**        **US-A- 5 196 459**
**US-A- 5 269 958**        **US-A- 5 653 963**
**US-A1- 2002 000 236**   **US-A1- 2003 026 765**
**US-A1- 2003 062 042**   **US-A1- 2004 102 768**
**US-A1- 2004 131 696**

• **PATENT ABSTRACTS OF JAPAN vol. 1997, no. 11, 28 November 1997 (1997-11-28) & JP 09 173926 A (MURATA MFG CO LTD), 8 July 1997 (1997-07-08)**
• **ANONYMOUS: "Care of dry skin: Dry Skin Relief Soothing Spray" INTERNET ARTICLE, [Online] XP002370047 Retrieved from the Internet: URL: http://www.eucerin.co.uk/uk_range/dry/ sooth_ spray.html> [retrieved on 2006-02-26]**

## Description

## Background to the invention

**Therapeutic Effects of Cooling the skin, chemical and electromechanical stimulation**

Reducing the skin temperature has been proven to have a therapeutic effect on itching and pain. [Fruhstorfer H, Hermanns M, Latzke L., Effects of Thermal Stimulation on Clinical and Experimental Itch. Pain 1986 Feb; 24(2): 259-69]. Pain and Itch are closely related sensations and are both transmitted by the same thin afferent nerve fibres, mainly C fibres. Cooling, In addition to anaesthetizing nerve endings, may act peripherally or centrally to inhibit pain or Itch [Fruhstorfer H, Hermanns M, Latzke L., Effects of Thermal Stimulation on Clinical and Experimental itch. Pain 1986 Feb; 24(2):259-69], [Bromm B., Scharein E., Darsow U. and Ring J., Effects of menthol and cold on histamine-induced itch and skin reactions In man. Neurosci. Lett,187 (1995) 157-160]. There is a distinct difference between freezing the outer surface of the skin and cooling it. The therapeutic effects referred to herein are due to cooling the outer surface of the skin, by few degrees below the normal 37°C, and not to cool it to a temperature close to or at 0°C. There are numerous inventions for treating sports injuries which involve using very cold cooling sprays of temperature, when utilized, is below or close to 0°C. Such sprays are capable of reducing the outer surface of the skin temperature from the normal 37°C to close to or at 0°C and have similar effect to applying ice cubes on the skin to reduce inflammation and bruising through vasoconstriction. One of the purposes of the present invention is to cool the outer surface of the skin and not to freeze it or cool It below 5°C. Applying very cold spray, the temperature of which is below 0°C which may cause the outer surface of the skin to freeze or reach a temperature close to or at 0°C will work against the intended processes of the present invention and is not within the scope of the present invention.

Mast cells are broken by direct damage and by abnormal tissue chemistry including antigens and peptides released by excited unmyelinated sensory fibres. They release histamine and proteolytic enzymes such as chymotrypsin. Proteolytic enzymes produce intense Itching. Such enzymes are known to function best at an optimum pH range. Alkaline solutions reduce itching [Madden EJ. Itch, J Pain Symptom Management 1986 Spring;1(2): 97-9] e.g. sodium bicarbonate, added to bath water has been found beneficial in atopic eczema [Litt JZ: Topical treatments of itching without corticosteroids; Bemhard JD(ed): Itch Mechanisms and management of Pruritis. New York, McGraw-Hill, 1994,pp 383-397]. In this respect, a composition with pH levels ranging from 7.01 - 12.6 may result In a similar effect on the relief of itching. Applying alkaline solutions Is regarded as a laborious home therapy and It Is difficult precisely to adjust the pH level to an optimum therapeutic level.

Transcutaneous Electrical Nerve Stimulation (TENS) and Mechanical Stimulation are also proven to have therapeutic effects on itching and pain [Ekblom A, Fjellner B, Hansson P. The influence of mechanical vibratory stimulation and transcutaneous electromechanical nerve stimulation on experimental pruritus induced by histamine, Acta Physiol Scand, 1984; 122: 361-7]. The applied pulse frequency seems to have an effect on the therapeutic results, Low-frequency TENS (2Hz) significantly reduced itch and the rhythmic muscle contractions induced by this type of stimulation were shown to have a pain-reducing effect. [Ekblom A, Fjellner B, Hansson P. The influence of extrasegmental mechanical vibratory stimulation and transcutaneous electromechanical nerve stimulation on experimental pruritus induced by histamine, Acta Physiol Scand, 1984; 122: 361-7].

TENS has disadvantages. It is dependent on an electrical power supply, it starts to relieve pain or itch only after continuous use for thirty minutes on average. It must not be used by patients using a demand type pacemaker, It cannot be used everywhere on the body e.g. not on the neck, and it should not be used when driving or operating machinery. It is not suitable for use by pregnant women and patients who have heart disease or epilepsy. TENS is not suitable for use when the area required treating is large or covers the greater part of the body; it is more suitable for treating localised pain or itching affecting a specific point on the body or a small area. Consequently, to date, TENS has not come into clinical use on a wide scale; furthermore patient compliance Is not good.

## Disclosure of the Present Invention

According to the present invention there Is provided an aerosol apparatus containing a water-based composition, the viscosity of which composition is from 0.2 to 30 mPa.s at 20°C, which aerosol apparatus Is adapted in use to release cool water-based particles for the simultaneous topical application of thermal (cooling) and mechanical kinetic energy stimuli for skin or body tissue cooling and for the generation of pulses In the body respectively, which aerosol apparatus is arranged in use to release non-flammable, water-based particles at a pressure of from 0.5 to 11 bars at 20°C, which pressure Is created by using dimethyl ether or liquefied petroleum gas or a mixture of the two, to discharge said water-based particles at a rate of from 0.1 to 8 gm/sec at 20°C at a discharge angle of from 0.0° to 145° at 20°C, at a temperature which is higher than 0°C and lower than the ambient temperature and with a particle diameter of from 10 to 600 microns, which aerosol apparatus is used for, but not limited to, the treatment of human and veterinary diseases and symptoms of diseases.

Preferably the cool water-based particles which the aerosol apparatus of the present invention is adapted in use to produce have an alkaline composition and preferably a pH of from 7.01 -12.6, thereby also to apply chemical stimulus for skin or body tissue e.g. to counteract the effect of proteolytic enzymes and reduce the itching.

The present invention provides an aerosol apparatus containing a composition adapted in use simultaneously to apply thermal, chemical and mechanical kinetic energy stimuli, so as to effect skin or body tissue cooling, to deliver water-based particles with the required pH level on the skin or body tissue and to generate mechanical kinetic energy pulses in the body respectively, which aerosol apparatus Is used for, but not limited to, the treatment of human and veterinary diseases and symptoms of diseases. The aerosol apparatus and composition of the present invention is capable of achieving an efficacy far exceeding that of each stimulus I.e. cooling, mechanical or chemical acting independently. Moreover in the majority of cases the aerosol apparatus containing the composition produces instant central (CNS) and peripheral inhibition action within a few seconds of application e.g. to abolish or reduce itching and pain. The aerosol apparatus is suitable for use anywhere on the body, is suitable for all adults, including pregnant women, children and infants and Is free from side-effects. In addition to instantly relieving symptoms of diseases e.g. itching, pain, skin rashes, hives, erythema, exudation and dryness, inflammation, etc., the present Invention has been proven to be effective in treating cutaneous diseases e.g. atopic dermatitis, prickly heat, urticaria as well as relieving their symptoms.

The mechanical kinetic energy stimulus occurs when the aerosol apparatus releases particles which carry kinetic energy and impact the skin or tissues. The Impact pressure of each particle on the skin is converted to vibration or pulses in the skin or body tissues. There are many types of kinetic energy. The type utilised in the present invention is translational kinetic energy. It is the energy of a particle moving in space and is defined in terms of the particle's mass, m, and velocity, v:

$$KE = \frac{1}{2}mv^2$$

The quantity of kinetic energy generated Is directly proportional to the mass of the particle travelling in space and also directly proportional to the square of the velocity. Therefore the velocity of the particle has greater impact on the value of the KE than does the mass. The aerosol apparatus of the present invention contains a non-flammable composition and permits the release of non-flammable water-based particles at a pressure of from 0.5 to 11 bar at 20°C, preferably at a pressure of from 1.5 to 8 bar at 20°C; the particles travel freely in space at a given velocity and the majority impact the skin or body tissues to create pulses of varying frequency. The temperature of the water-based particles when utilised is greater than 0°C and lower than the ambient temperature. The pressure at which the water-based particles is released is such that the velocity of the particles gives rise to the optimum amount of kinetic energy which is sufficient to create an optimum pulse frequency so as to result in maximum therapeutic efficacy. The aerosol apparatus of the present invention is adapted on the one hand to prevent the particles to float freely and aimlessly in air and on the other hand to prevent the formation of a foam or sherbet-like spray as the particles must be directional, aimed to travel in space In a specific direction and to impact the skin or body tissues at a pressure. The number of particles released is dependent on the discharge rate which is selected to be from 0.1 to 8 gm/sec at 20°C, preferably from 0.2 to 5 gm/sec or from 0.4 to 3 gm/sec at 20°C. The angle of discharge affects the size of the skin area Impacted by the particles and is selected to be from 0.0° to 145° at 20°C, preferably from to 5° to 60° at 20°C. This permits the treatment of large areas of the body in one application and the possibility of treating within seconds as much or as little body surface area as required.

According to the present invention the diameter of the released particles is from 10 to 600 microns, preferably from 10-250 microns or from 15 to 300 microns In order that the particles possess adequate mass and velocity and to prevent inhalation of the particles.

The means for achieving the specified particle size, the specified discharge rate and the specified angle of discharge at a specific temperature will be apparent to one skilled in the art.

One possible means of achieving the required particle size, discharge rate and angle of discharge at 20°C is by selecting an appropriate aerosol valve, actuator and actuator insert from those currently available on the market and supplied by third parties in conjunction with the selected composition (with appropriate viscosity) contained in the aerosol apparatus which composition also determines the pressure within the aerosol apparatus at a specific temperature (if the composition includes a pressurized gas then the pressure of that gas within the aerosol apparatus will affect the overall pressure and will be taken in account), the valves, actuators and actuator inserts release the composition at the required discharge rate and angle of discharge after breaking it into the required particle size. The discharge rate may be determined e.g. by measuring the weight loss from the aerosol apparatus in a specified time e.g. 10 seconds at a specified temperature e.g. 20°C and repeating the process several times to calculate the average. The discharge rate in gm/sec at the specified temperature can then be readily calculated. To measure the particle size, a spray particle analyzer may be used e.g. one which uses Light Interaction Method e.g. laser diffraction. There are several such particle analyzer instruments on the market supplied by third parties to measure such particles diameter e.g. Spraytec- Spray particle analyzer supplied by Malvern Instruments Ltd of Enigma Business Park, Grovewood Rd, Malvern, WR14 1XZ, UK or Microtrac S3500 supplied by Microtrac Inc. of 12501A- 62nd St, North Largo, FL 33773, USA. To measure the viscosity, Viscolab 4000 System from Cambridge Applied Systems Inc, 10 Presidents Landing, Medford, MA 02155-5148, USA may be used or AMVn Automated Microviscometer from Anton Paar Ltd, 13 Harforde Court, Hertford, SG13 7NW, UK.

The required cooling level is achieved by dimethyl ether or liquefied gas or a mixture of the two. When the composition is released from the aerosol apparatus into the atmosphere, the latent heat of transformation of dimethyl ether or the liquefied gas or a mixture of the two from liquid to gas is absorbed from the said composition thus cooling the water-based particles to a temperature which when utilized is greater than 0°C and lower than the ambient temperature. Cooling ingredients e.g. menthol, menthyl lactate may be added to enhance the cooling effect on the skin. The discharge rate decides the level of skin cooling as the level of skin cooling is directly proportional to the discharge rate.

The pressure of from 0.5-11 bar at 20°C is achieved by using dimethyl ether or liquefied petroleum gas or a mixture of the two. Pressurized gases e.g. Nitrogen or Carbon Dioxide may be added as well to help achieve the adequate pressure level.

When the water-based particles impact the skin or body tissues, the mechanical kinetic energy stimulus will then be converted into pulses having specific therapeutic pulse frequency. The pulses travel via A fibres to cause Inhibition of dorsal hom cells and to regulate amplifying effects of interneural circuits thereby to lead to local segmental suppressive effects on itching and pain traffic through the CNS (Central Nervous System). In 1965 Meizsck and Wall proposed the "gate control" theory to explain why mechanical stimulation reduces pain sensation. They suggested that impulses in afferent large-diameter myelinated A-fibres, activated by touch, pressure or vibratory stimulation, modulate and inhibit simultaneous Impulses In C-fibres reaching the spinal cord i.e. A-fibre input closes the "gate" for C-fibres input of pain at spinal level.

When the aerosol apparatus of the present invention is operated it produces, for the duration of use e.g. while the actuator button is pressed, a discharge of particles, which is continuous, not intermittent. Intermittent or automatic "stop-start" discharge at a fixed ratio (as detailed in EP-A-1 195 173, Daizo Corporation, 10 April 2002) or at random, is not desirable and works against the intended processes of the present invention and is not within the scope of the present invention. The transfer of mechanical kinetic energy via the released particles must be continuous and not "stop-start" in order to achieve the desired continuous mechanical stimulus effects i.e. continuous impact pressure and pulses in the skin or body tissues for the duration of use.

To achieve the desired effect in accordance with the present invention, the skin or tissues should be bare. A sufficiently thin and sufficiently perforated cover e.g. certain type of ladies' stockings may still allow transfer of the kinetic energy and may be used. If the skin Is covered by a dressing or clothing the mechanical kinetic energy will be absorbed by that dressing or clothing and will be prevented from being transmitted further to the skin to generate the desired pulses in the body. Excessive hair (or fur in case of animals) should preferably be shaved to allow transfer of kinetic energy to the skin.

The present invention permits the flexibility of altering the transmitted pulse frequency and amplitude resulting from the mechanical kinetic energy stimulus acting on the skin. The pulse frequency is dependent on the speed of the released particles which is dependent on a pressure within the aerosol apparatus of from 0.5 to 11 bar at 20°C. The pulse amplitude is dependent on the mass of the released particles which is dependent on the viscosity of the composition (from 0.2 to 30 mPa.s at 20°C, preferably from 0.3 to 15 mPa.s. at 20°C) and the particle size (from 10 to 600 microns, preferably from 10-250 microns or from 15-300 microns). The pulse frequency in Hz and the amplitude of the released particles can be measured by an accelerometer fitted with an appropriate sensor suitable for micro measurements. Different diseases and symptoms seem to respond better to specific pulse frequencies and amplitudes. The optimum therapeutic pulse frequency for specific diseases are decided by clinical studies. Therefore by adjusting the aerosol apparatus pressure, viscosity and particle size, the treatment of specific diseases can be targeted. By selecting the appropriate aerosol apparatus according to the present invention, each patient can control the dosage that best suits their needs by controlling the number and duration of applications until symptoms ease. A factor which only the patient can determine, especially when the symptoms are subjective e.g. pain or itching.

The water-based particles released at the specified pressure mechanically cleanse and debride the skin lesions, if available and prevent serum and crust from accumulating. They also help in macerating vesicles.

The particles released from the aerosol apparatus according to the present invention travel in air for impacting the body and for safety reasons the particles must be non-flammable i.e. the flammable content of the composition contained in the aerosol apparatus must not exceed 45% by weight in accordance with BS3914, This safety feature allows the users to use It safely anywhere.

The water content is from 36 % to 92%, preferably from 45% to 75% to reduce any proposed toxic content and in order to prevent the water-based particles from freezing or reaching low temperatures close to 0°C when going through the cooling process. Such low temperatures are not desirable as the purpose of the present invention is to cool the skin and not to freeze it or cool it below 5°C. The high water content allows the aerosol apparatus to be used anywhere on the body including the face, due to the low toxicity. The water also evaporates slowly off the skin or tissue surface resulting in an increase in the cooling effect and an increase in the duration of the cooling effect, which are therapeutically desirable.

High water content permits skin and lesion hydrotherapy which has an important therapeutic role in dermatology. Many skin diseases are caused by dry skin. Skin is not dry because it lacks oil, but because it lacks water. Bathing and showering help hydrate the skin, however hot baths removes the natural skin oils more quickly and cool baths are less comfortable. Applying wet towels Is a laborious home therapy and is difficult to apply to large areas of the body. Applying the pressurized water-based particles of the present invention to skin and to lesions results in hydration of the skin and deep hydration of the lesions (due to the skin disorder and the state of the stratum comeum lesions will more readily absorb water than healthy skin.) Hydration is important to compensate for the transepidermal water loss, especially for itchy xerotic skin. Hydrotherapy reduces the transepidermal water loss and blood flow associated with skin irritation. It accelerates the healing of underlying skin properties. Water has known hygroscopic characteristics and may therefore increase the capacity for intracellular moisture retention. Additionally water Improves the barrier function and reduces Inflammation. The cool particles have a vasoconstriction, antl-inflammatory effect on inflamed skin or tissues.

According to the present invention, the simultaneous thermal (cooling) and mechanical kinetic energy transferred to the skin, through the released particles from the said apparatus which impact the human or animal body, has the function of cooling, revitalising the body, combating exhaustion and fatigue and prolonging sport performance. The mechanical kinetic energy stimulus has the effect of magnifying the cooling stimulus on the skin and the heat relief of the body which Is superior to the effect of cooling alone. Other functions may include but are not limited to, instant relief of menopausal symptoms, First Aid usages e.g. resuscitating a person or animal which has fainted or lost consciousness or the treatment of bums.

According to the present Invention, solvents e.g. alcohol or dimethoxymethane (methylal) may be added to the composition contained in the aerosol apparatus to stabilise the composition used In the aerosol apparatus, to adjust the vapour pressure or to assist in producing a one-phase mixture. The amount of solvent included is preferably limited to up to 15 % by weight of the composition. The latent heat of evaporation of water is relatively high compared with other solvents therefore reducing the solvent content and increasing water content results In enhanced cooling In the presence of dimethyl ether or liquefied petroleum gas or a mixture of the two.

The composition contained in the aerosol apparatus of the present invention may contain, but not limited to any one or more of the following:

An analgestic agent, an anti-inflammatory agent, an anti-pruritic agent, an antiseptic agent, a disinfectant, a germicide, an antibiotic, an antifungicide, an emulsifier, an anti-oxidant, a corrosion inhibitor, a fragrance, a cooling agent, an aromatic alcohol e.g. menthol, camphor, a stabilizing agent, a solubilizing agent, a pH adjusting agent and a skin conditioning agent.

The following examples are given to further illustrate the present invention. One embodiment of the present invention is as detailed below. However the invention Is not limited thereto. An aerosol dispenser is produced containing a cooling water-based composition. The pressure inside the aerosol dispenser is created by using dimethyl ether or liquefied petroleum gas or a mixture of the two. Pressurized gas e.g. Nitrogen or Carbon Dioxide may be added to achieve the required pressure. The cool water- based particles, released from the dispenser, travel at a reasonable speed in space in the form of a mist of fine particles from 30-70 microns approximately.

Example 1:

The aerosol dispenser contained the following :

| INGREDIENTS | % BY WEIGHT |
| --- | --- |
| Water | 60% |
| Methylal | 3.1% |
| Ethanol | 2.5% |
| Alpha-tocopherol | 0.5% |
| Fragrance | 0.01% |
| Ethanolarnine Borate | 0.25% |
| Dimethyl Ether | 31% |
| Liquefied petroleum gas | 2.64% |

(continued)

| INGREDIENTS | % BY WEIGHT |
|---|---|
| Pressure at 20°C : 4.5 bar<br>Particle size : 50 microns<br>Discharge rate at 20°C: 1.3 gm/sec<br>Discharge angle at 20° C : 25°<br>pH : 9.2<br>Viscosity at 20°C : 0.71 | 100% |

All the studies detailed below were conducted in accordance with EU guidelines on Good Clinical Practice and the Declaration of Helsinki. The clinical studies were approved by the local Ethics Committee. The new intervention of the present invention, which is the aerosol apparatus containing the composition, as detailed in Example (1) shall be referred to in the clinical studies as N1.

The patients taking part in the clinical studies were given free kits of the aerosol apparatus of the present invention and a set of Instructions detailing method of use as follows: 1) Remove clothing of the area of application. 2) If the face needs to be treated, the eyes should be closed or masked (for babies or small children) 3) Hold the apparatus at a distance of 10 cm - 20 cm approximately. 4) Press the apparatus's actuator to release the water-based particles. 5) Apply for a few seconds or as needed then leave the treated areas to dry untouched. Repeat as many times as needed until the symptoms ease. 6) In chronic cases and in order to speed the healing process, apply a daily regimen of four times per day, once before bedtime until symptoms reduce or disappear.

Clinical Study 1:

94 Patients (n=52 women: n=42 men) of mean age 32.51 $\pm$ 21.48 years took part In an Open Label clinical study to assess the effectiveness of N1 in abolishing or reducing pruritus. The patients suffered severe itching related to atopic dermatitis (n= 22), Contact dermatitis (n=6), Psoriasis (n=8), Urticaria (n=15), Xerosis (n=6), Prickly heat (n=8), Dermal allergies (n=7), Hand eczema(n=7), Poison ivy(n=3), Lichen planus (n=8)and insects bite (n=4). The patients were given a sample of N1 and a questionnaire to take home for three days self-monitoring period. The patients were requested to return on day 4 with completed questionnaire. The questionnaire includes a 10 cm Visual Analogue Scale (VAS), graded from 0 (no Itch) to 10 (severe itch). The patients record the itching intensity after applying N1 following each of three severe Itch attacks (Vas=10) and the length of the itch-free period following each application on a scale from under one hour to 72 hours ( 3 days), The efficacy of N1 is evaluated by the patients in abolishing, reducing itching, effect on their sleep pattern, whether it is more effective than the traditional treatment they used in the past in abolishing, reducing itching and whether they experienced any adverse events.

All patients started from itch level (VAS=10). 82% (n=77) of patients experienced VAS of 0(no itch) within few seconds after N1 application i.e. instant rellef from itch, 8.5 % (n=8) experienced VAS of 1-5 (low-medium itch), 9.5% (n=9) experienced VAS of 6-10 (medium-severe itch).

27.6% (n=26) suffered one severe itch attack and remained itch free for the three day monitoring period after applying N1 . 72% ( n=68) suffered second severe itch attack, 64.8% ( n=61) suffered a third severe itch attack during the same monitoring period.

The Itch free period after successfully applying N1 and breaking the itch-scratch cycle, ranged from two hours to seventy two hours (the full three days of self-monitoring) with a mean of 32.03$\pm$27.25 hours, The longest itch free duration was experienced by contact dermatitis cases (48$\pm$18.59 hours), followed by prickly heat cases (47.79$\pm$26.95 hours). The least itch free duration was recorded by psoriasis cases (5.85$\pm$3 hours). The difference in breaking itch cycle duration between the eleven groups was statistically significant where $F^{11}_{186}$ =6.672, P=0.0001

89 patients using N1 entered their sleep pattern on questionnaire. 73% (n=65) slept through the night. 19% (n=17) experienced slight sleep disturbance and 7.8% (n=7) experienced severe disturbance.

87 patients entered their anti-pruritic drug preference in the questionnaire. 82.7% (n=72) stated N1 is more effective than conventional drugs they used in the past and is easier to use, 9% (n=8) stated that the conventional antl-pruritic treatment is more effective. 8 % (n=7) found no difference between the two. No adverse events were recorded.

Clinical Study 2:

125 Patients took part In a comparative, randomized, single (observer) blind, controlled clinical study. The study was to compare, over a period of two weeks, the efficacy and safety of N1 and Hydrocortisone 1% in (1) treatment of Atopic Dermatitis (2) Treatment of symptoms of Atopic Dermatitis, also common to most cutaneous disorders and some systemic disorders (i) Dryness (II) Lichenification (iii) Cracking (Iv) Erythema (v) Exudation (vi) Excoriation (vii) Itching. The patients were randomized to Group (1) 61 patients (males n=25 (41%), mean age of which Is $31.45\pm14.95$ years and females n=36 (59%), mean age of which is $29.73\pm16.47$ years) who were treated with N1 for two weeks and Group (2) 64 patients (males n= 26 (40%), mean age of which is $30.26\pm14.65$ years and females n=38 (60%), their mean age of which is $29.48\pm14.64$) who were treated with Hydrocortisone 1% for two weeks. The age difference for both groups of males and females was statistically Insignificant t=0.551, P=0.582 and t=1.307, P=0.194 respectively. The overall age of the N1 group ranged from 6 to 59 years with an overall mean of $30.44\pm17.96$ years in comparison to a similar range of age but with a mean of $28.03\pm18.1$ years for the hydrocortisone group with no statistical significance t=0.197, P=0.844. male and females were similarly distributed among the two studied groups where males constituted 40% approximately of each of the two groups. The similar distribution allowed for a matching analysis with no statistical difference where Chi-square test=0.001, P=0.976.

The scoring system used to evaluate the results of both groups is "Six area, six sign atopic dermatitis (SASSAD) severity score [Jones JB. Six area, six sign atopic dermatitis (SASSAD) severity score : A simple system for monitoring disease activity in atopic dermatitis. Br J Dermatol. 1996; 135 (Suppl 48); 25-30] The six signs of atopic dermatitis were evaluated in each patient of the N1 and Hydrocortisone Groups, in six areas of the body. The signs are: Dryness, lichenification, cracking, erythema , exudation and excoriation . the six areas of the body are head, neck , trunk, arms, hands, legs and feet. The severity of the lesions are assessed as 0, 1, 2 and 3 for no lesion, mild, moderate and severe lesions respectively for each sign in each body area resulting In a score of maximum 18 for each sign. The six signs were evaluated objectively by the investigating physicians, Itching being a subjective symptom was evaluated by the patient.

The atopic dermatitis diagnosis was based on [Hanifin J.M., Rajka G. Diagnostic Features of Atopic Dermatitis, Acta Derma (Stock) Suppl. 92:44-47, 1980]. SASSAD score for each patient was evaluated at baseline, at end of week 1 and at the end of week 2 following treatment by either N1 or Hydrocortisone 1%,

Table I reveals the mean SASSAD score of the two studied groups before and after one and two weeks of N1 and hydrocortisone treatment. No statistical difference was observed for SASSAD score at baseline of both N1 and hydrocortisone groups, where the mean scores were $17.53\pm7.85$ and $17.52\pm7.37$ respectively t=0.132, P=0.99. However, after one week of regular use of both medication, a statistical significant difference was detected where the mean SASSAD score of the N1 Group was significantly less than the Hydrocortisone Group ($4.04\pm6.1$ and $12.82\pm8.34$ respectively) where t= -9,15, P=0,0001.

**Table I: Mean SASSAD score before and after N1 and Hydrocortisone use in the two groups.**

|  | N1 | | | Hydrocortisone | | | t | P |
|---|---|---|---|---|---|---|---|---|
|  | n | Mean | SD | n | Mean | SD |  |  |
| Score at baseline | 61 | 17.53 | 7.85 | 64 | 17.52 | 7.37 | 0.13 | 0.99 |
| Score at end of week one | 61 | 4.04 | 6.1 | 64 | 12.82 | 8.34 | -9.15 | .0001 |
| Score at end of week two | 36 | 2.86 | 5.73 | 55 | 8.4 | 6.0 | -6.27 | .0001 |

25 Patients of the N1 group were cleared from atopic dermatitis (SASSAD score=0) compared to only 9 patients (3.81%) of the hydrocortisone group. The rest of the patients continued their treatment for a second week, their SASSAD score declined to $2,86\pm5.73$ and $8.4\pm6$ for N1 and hydrocortisone groups respectively. The difference between both scores was statistically significant where t=-6.27, P=0.0001.

The decline in the SASSAD scores of both groups throughout the treatment period (two weeks) is shown in Table II. The N1 group have shown a decline in their SASSAD score from $17.53\pm7.85$ before treatment to $4.04\pm6.16$ after one week of treatment, this decline was found to be statistically significant where t=20.12, P=0.0001, 36 patients have continued their treatment for a second week to reach a score of $2.86\pm5.73$ with also a statistical significant drop where t=5.41, P=0,0001.

A similar result was observed in the Hydrocortisone group where a significant decline In their score before and after one week of treatment from a score of $17.51\pm7.37$ to a score of $12.52\pm8.34$ where t=8.83, P=0.0001. The fifty five patients who continued their treatment for the second week showed a further score decline to $8.4\pm6$, this decline was statistically significant where t=10.39, P=0.0001.

A SASSAD score of 0 or 1 Is considered as a cleared lesion. Accordingly it was found that in N1 group after one week of treatment, 23 patients (37.7%) scored 0 and 2 cases (3.2%) scored 1; therefore 42.7% of the N1 group were cleared of atopic dermatitis after Week One compared to 14% in the Hydrocortisone group. At the end of Week Two 24 patients of the N1 group (39%) were cleared of atopic dermatitis compared to 4 patients in the Hydrocortisone group (6.3%).

The study clearly shows that after two weeks treatment 81.4% of the N1 Group were cleared of Atopic dermatitis compared to 11% of the Hydrocortisone Group.

**Table II: Mean change in SASSAD score during the two weeks follow up period in the N1 and Hydrocortisone Groups**

| SASSAD Score | N1 | | | | | Hydrocortisone | | | | |
|---|---|---|---|---|---|---|---|---|---|---|
| | n | Mean | SD | Paired test | P | n | Mean | SD | Paired t test | P |
| Base | 61 | 17.5 | 7.85 | | | 64 | 17.5 | 7.37 | | |
| Week 1* | 61 | 4.04 | 6.16 | 20.1 | .0001 | 64 | 12.5 | 8.34 | 8.83 | .0001 |
| Week 2** | 36 | 2.86 | 5.73 | 5.41 | .0001 | 55 | 8.4 | 6 | 10.39 | .0001 |
| * Paired t test is calculated between the base score and the first week score. <br> ** Paired t test is calculated between the base score and the second week score | | | | | | | | | | |

Analysis of individual signs of atopic dermatitis in the two groups over the two weeks follow up period has revealed the following findings

Dryness

Table III details the mean dryness score comparison between the two groups. A minor difference existed between the two groups at baseline, however this difference increased after one and two weeks of treatment. The N1 group registered a greater drop of the mean score after one week from 3.66± 2.56 to 1.31±1.8 and reached 0.76±1.4 after the second week This decline was statistically significant where Wilcoxon signed rank test=-9.04, P=.000 and=-5.87. P=.000 after one and two weeks respectively. Fig (1) shows the mean dryness score comparison between the two groups.

Table III: Mean Dryness score before and after N 1 and Hydrocortisone use in each group

| | N1 | | | Hydrocortisone | | | Mann-Whitney U test | P |
|---|---|---|---|---|---|---|---|---|
| | n | Mean | SD | n | Mean | SD | | |
| Base score | 61 | 3.66 | 2.56 | 64 | 4.32 | 1.98 | -3.45 | .001 |
| Week 1 score | 61 | 1.31 | 1.81 | 64 | 2.9 | 2.1 | -7.16 | .000 |
| Week 2 score | 36 | .76 | 1.4 | 55 | 2.13 | 1.7 | -6.29 | .000 |
| | Wilcoxon sign rank test | | P | Wilcoxon sign rank test | | P | | |
| **Week 1-Base** | -9.04 | | .000 | -7.73 | | .000 | | |
| **Week 2-Week 1** | -5.87 | | .000 | -7.05 | | .000 | | |

Lichenification

Variation in lichenification score in the N1 and hydrocortisone groups are detailed in table IV, The score has declined significantly from baseline in both groups, yet the decline was more prominent in the N1 group where the mean score has declined from 1.83±2.1 before treatment to 0.65±1.3 after week one and 0.46± .91 after week two. In the second group the score has declined from 2.44±1.7 before treatment to 1.55±1.9 after week one and .82±1.3 after week two.

Wilcoxon Sign ranks test= -7.54,P=.000, and =-3.86, P=.000 for the first group after week one and week two and =-6.27,P=.000 and =-4.27,P=.000 for the second group after week one and week two. Fig (2) shows the mean score of lichenification in each group throughout the study period.

**Table IV: Mean Lichenification score before and after N 1 and Hydrocortisone use in each group.**

| | N1 | | | Hydrocortisone | | | Mann-Whitney U test | P |
|---|---|---|---|---|---|---|---|---|
| | n | Mean | SD | n | Mean | SD | | |
| Base score | 61 | 1.83 | 2.1 | 64 | 2.44 | 1.7 | -3.5 | .000 |
| Week 1 score | 61 | .65 | 1.3 | 64 | 1.55 | 1.9 | -4.67 | .000 |
| Week 2 score | 36 | .46 | .91 | 55 | .82 | 1.3 | -2.01 | .045 |
| | Wilcoxon sign rank test | | P | Wilcoxon sign rank test | | P | | |
| **Week 1-Base** | -7.54 | | .000 | -6.27 | | .000 | | |
| **Week 2-Week 1** | -3.86 | | .000 | -4.27 | | .000 | | |

Cracking

Cracking responded to both interventions. The difference in score between the two groups at baseline was small. However, N1 group score at end of week one and two was half of that for Hydrocortisone(0.21±.78 and 0.44±.84 after week one and 0.1±.16 and 0.21±.61 after week two in both groups respectively). The decline in score throughout the treatment was statistically significant In both groups (Wilcoxon Sign ranks test= -6.15,P=.000, and =-2.97, P=.003 for the N1 group after one and two weeks treatment and =-6.29,P=.000 and =-3.59,P=.000 for the hydrocortisone group after the first and second week treatment respectively) as demonstrated in Table V. Fig(3) shows the mean score of cracking in both groups throughout the study period.

Table V: Mean Cracking score before and after N 1 and Hydrocortisone use in both groups.

| | N1 | | | Hydrocortisone | | | Mann-Whitney U test | P |
|---|---|---|---|---|---|---|---|---|
| | n | Mean | SD | n | Mean | SD | | |
| Base score | 61 | .95 | 1.7 | 64 | 1.17 | 1.1 | -3.31 | .001 |
| Week 1 score | 61 | .21 | .78 | 64 | .44 | .84 | -3.32 | .001 |
| Week 2 score | 36 | .11 | .16 | 55 | .21 | .61 | -2.72 | .006 |
| | Wilcoxon sign rank test | | P | Wilcoxon sign rank test | | P | | |
| **Week 1-Base** | -6.15 | | .000 | -6.29 | | .000 | | |
| **Week 2-Week 1** | -2.97 | | .003 | -3.59 | | .000 | | |

Erythema

The mean score of erythema was significantly higher In the N1 group than the hydrocortisone group at base line (4.6±2.3 and 3.6±2.5 for N1 and hydrocortisone groups respectively), however the N1 score was significantly lower after week one and week two, where Mann Whitney-U test=-8,21,P=.000 and -5.17,P=.000 for the difference at end of first and second week respectively. The decline in score in both groups at end of week one and week two are statistically significant as shown In Table VI. Where Wilcoxon sign ranks tests For the N1 group =-8.96,P=.000 and - 3.04, P=.000 at end of week one and week two respectively, and for the hydrocortisone group=-4,98, P=.000 and -6.46, P=.000 at end of week one and week two respectively. Fig(4) shows the mean score of erythema in both groups throughout the study period

**Table VI: Mean Erythema score before and after N1 and Hydrocortisone use in both groups.**

| | N1 | | | Hydrocortisone | | | Mann-Whitney U test | p |
|---|---|---|---|---|---|---|---|---|
| | n | Mean | SD | n | Mean | SD | | |
| Base score | 61 | 4.6 | 2.3 | 64 | 3.6 | 2.5 | -2.58 | .010 |
| Week 1 score | 61 | .72 | 1.57 | 64 | 2.9 | 2.5 | -8.21 | .000 |

(continued)

| | N1 | | | Hydrocortisone | | | Mann-Whitney U test | p |
|---|---|---|---|---|---|---|---|---|
| | n | Mean | SD | n | Mean | SD | | |
| Week 2 score | 36 | .68 | 1.6 | 55 | 1.8 | 1.98 | 5.17- | .000 |
| | Wilcoxon sign rank test | | P | Wilcoxon sign rank test | | P | | |
| **Week 1-Base** | -8.96 | | .000 | -4.98 | | .000 | | |
| **Week 2-Week 1** | -3.04 | | .000 | -6.46 | | .000 | | |

Exudation

The difference in exudation scores for both groups at baseline was not statistically significant, Where Mann Whitney -U test =.626, P=.531. At the end of week one, N1 group reallzed a significantly less mean score than the hydrocortisone group (0.54±1.3 and 2.5±2.4 respectively). At the end of week two as shown in Table VII the significant difference remained between both groups (0.55±1.4 and 1.67±1.79 respectively). The mean score decline In both groups was found to be statistically significant where Wilcoxon sign ranks test=-8.58,P=.000 and=-2.21, P=.027 for N1 group at the end of week one and week two respectively and it was -4.98,P=.000 and=-6.64, P=.000 for Hydrocortisone group at the end of week one and week two respectively. Fig(5) shows the mean score of exudation in both groups throughout the study period.

**Table VII : Mean Exudation score before and after N1 and Hydrocortisone use in both groups.**

| | N1 | | | Hydrocortisone | | | Mann-Whitney U test | P |
|---|---|---|---|---|---|---|---|---|
| | n | Mean | SD | n | Mean | SD | | |
| Base score | 61 | 3.3 | 2.17 | 64 | 3.18 | 2.6 | -.626 | .531 |
| Week 1 score | 61 | .54 | 1.3 | 64 | 2.5 | 2.4 | -7.91 | .000 |
| Week 2 score | 36 | .55 | 1.4 | 55 | 1.67 | 1.79 | -5.2 | .000 |
| | Wilcoxon sign rank test | | P | Wilcoxon sign rank test | | P | | |
| **Week 1-Base** | -8.58 | | .000 | -4.98 | | .001 | | |
| **Week 2-Week 1** | -2.21 | | .027 | -6.46 | | .000 | | |

Excoriation

Table VIII shows that the difference in the mean excoriation score between both groups is statistically Insignificant at baseline, where Mann Whitney - U test = -1.31, P=.191, however it became significant, where Mann Whitney -U test=-6.43, P=.000 and -5.81, P=.000 at the end of week one and week two respectively. N1 group showed a significant decline in the mean score from 3.16±2.4 at baseline to 0.54±1.16 after one week and 0.51±1.3 after the second week, this decline was statistically significant where Wilcoxon Sign ranks test = -8.01, P=.000 at the end of week one and -2.85, P=.005 at the end of week two. Similarly the decline in the mean score in the hydrocortisone group was also statistically significant where Wilcoxon sign rank test= -5.51, P=.000 and - 3.62 P=.000 for week one and week two respectively. Fig(6) shows the mean score of excoriation In both groups throughout the study period.

**Table VIII: Mean Excoriation score before and after N 1 and Hydrocortisone use in both groups.**

| | N1 | | | Hydrocortisone | | | Mann-Whitney U test | P |
|---|---|---|---|---|---|---|---|---|
| | n | Mean | SD | n | Mean | SD | | |
| Base score | 61 | 3.16 | 2.4 | 64 | 2.7 | 2.5 | -1.31 | .191 |
| Week 1 score | 61 | .54 | 1.16 | 64 | 1.97 | 1.99 | -6.43 | .000 |
| Week 2 score | 36 | .51 | 1.3 | 55 | 1.6 | 1.58 | -5.81 | .000 |

(continued)

|  | Wilcoxon sign rank test | P | Wilcoxon sign rank test | P |  |  |
|---|---|---|---|---|---|---|
| **Week 1-Base** | -8.01 | .000 | -5.51 | .000 |  |  |
| Week 2-Week 1 | -2.84 | .005 | -3.62 | .000 |  |  |

Itching

Due to the subjective nature of this symptom, all the patients were given VAS (Visual analogue scale graded 0=no itch to 10=severe itch) to complete at home twice daily, once in the morning and once in the evening, on the fifth, sixth and seventh day, after allowing for a washout period of four days. Table IX clarify the mean VAS score in both groups after treatment with their respective intervention. The N1 group has experienced a significant decline in their VAS score throughout the 5th, 6th and 7th days where paired t test for the decline in the 5th day =6.96, P=0.001 and for the 6th day=6.74, P=0.001 and for the 7th day=2.76, p=0.006. The hydrocortisone group VAS score has declined In the 5th day from $8.01 \pm 1.65$ in the morning to 7.69 in the evening paired t=7.69, P=0.001, in the sixth day paired t=2.35, P=0.020, while in the 7th day, VAS score has declined from $6.63 \pm 1.93$ to $6.58 \pm 1.97$ and this minimal decline was statically insignificant where paired t=0.86, P=0.386.

Comparison of the efficacy of N1 and hydrocortisone as an anti-pruritic treatment in both groups is illustrated in Table X. The mean VAS of the N1 group after using N1 has reached $2.53 \pm 1.26$ in the morning of the fifth day of treatment and then dropped to $0.92 \pm 1.57$ in the evening of the seventh day, this drop is found to be statistically significant where paired t test=20.998, P=0.0001. However in the hydrocortisone group, the mean VAS has reached in the morning of the fifth day of their hydrocortisone treatment $8.01 \pm 1.65$, and then dropped to $6.58 \pm 1.97$, this drop Is similarly statistically significant where paired t=20.07, P=0.001, but it should be considered also that the VAS achieved by the second group was significantly worse than that of the first group N1 where t = -32.79, P=0.0001 for the 5th day in the morning and t =-27.95, P=0.001 for the 7th day In the evening

The itch free duration in the 3 day self-monitoring period after using N1 was a mean of $12.01 \pm 11.89$ hours. The corresponding figure for the Hydrocortisone group was a mean of $1.76 \pm 1.21$ hours.

**Table IX: Visual analogue scale (VAS) recordings at the 5th, 6th, and 7th day, morning and evening, in both groups.**

| VAS | Group I N1 | | | | | Group II Hydrocortisone | | | | |
|---|---|---|---|---|---|---|---|---|---|---|
|  | n | Mean | SD | Paired t test | P | n | Mean | SD | Paired t test | P |
| **Day 5** Morning | 61 | 2.53 | 1.26 | 6.96 | .001 | 64 | 8.01 | 1.65 | 7.69 | .001 |
| *Evening* | 61 | 2.29 | 1.31 |  |  | 64 | 7.69 | 1.76 |  |  |
| **Day 6** Morning | 61 | 1.79 | 1.17 | 6.74 | .001 | 64 | 7.17 | 1.74 | 2.35 | .020 |
| Evening | 61 | 1.52 | 1.31 |  |  | 64 | 7.06 | 1.78 |  |  |
| **Day 7** Morning | 61 | 1.09 | 1.55 | 2.76 | .006 | 64 | 6.63 | 1.93 | 0.86 | .386 |
| Evening | 61 | 0.92 | 1.57 |  |  | 64 | 6.58 | 1.97 |  |  |

**Table X: Comparison between VAS recordings of the 5th day, morning and 7th day, evening in both groups**

| VAS | Group I N1 | | | Group II Hydrocortisone | | | | |
|---|---|---|---|---|---|---|---|---|
|  | n | Mean | SD | n | Mean | SD | t test | P |
| 5th day Morning | 61 | 2.53 | 1.26 | 64 | 8.01 | 1.65 | -32.79 | .0001 |
| 7th day Evening | 61 | .92 | 1.57 | 64 | 6.58 | 1.97 | -27.95 | .001 |
|  | Paired t test=20.98 P=.0001 | | | Paired t test=20.07 P=.001 | | | | |

Example 2:

The aerosol dispenser contained the following ;

| INGREDIENTS | % BY WEIGHT |
|---|---|
| Water | 57.0% |
| Methylal | 4.69% |
| Ethanol | 3.3% |
| Menthol | 1.7% |
| Alpha-tocopherol | 0.05% |
| Fragrance | 0.01% |
| Dimethyl Ether | 26.25% |
| Liquefied petroleum gas | 7.0% |
| | |
| Pressure at 20° C: 4.8 bar Particle size : 70 microns Discharge Rate at 20°C : 0.9 gm/sec Discharge angle at 20° C : 30° pH: 6.9 Viscosity at 20°C : 0.73 | 100% |

Clinical Study 3 :

The new Intervention, which is the aerosol apparatus of the present invention, as detailed in Example 2 shall be referred to in the clinical studies as N2.

87 patients (n=47 women: n=40 men) of mean age 29.87 $\pm$ 19.64 years took part In an Open Label clinical study to assess the effectiveness of N2 in abolishing or reducing pain. The patients suffered severe pain related to Osteoarthritis (n= 26), Back pain (n=20), Shingles (n=9), Burning (n=10), Multiple Sclerosis (n=7), Pulled ligaments (n=7), Post operative pain (n=8). The patients were given a sample of N2 and a questionnaire to take home for three days self-monitoring period. The patients were requested to return on day 4 with completed questionnaire. The questionnaire includes a 10 cm Visual Analogue Scale (VAS), graded from 0 (no pain) to 10 (severe pain). The patients record the pain intensity after applying N2 following each of three severe pain attacks (Vas=10) and the length of the pain free period following each application on a scale from under one hour to 72 hours (3 days). The efficacy of N2 is evaluated by the patients in abolishing, reducing pain, effect on their sleep pattern, whether it is more effective than the traditional treatment they used in the past in abolishing or reducing pain and whether they experienced any adverse events.

All patients started from pain level (VAS=10). 78% (n=68) of patients experienced VAS of 0(no pain) within few seconds after N2 application i.e. instant relief from severe pain (VAS=10), 11.4 % (n=10) experienced VAS of 1-5 (low-medium pain), 10.3% (n=9) experienced VAS of 6-10 (medium-severe pain).

13.8% (n=12) suffered one severe pain attack and remained pain free for the three day monitoring period after applying N2 . 86.2% (n=75) suffered second severe pain attack, 71% (n=62) suffered a third severe pain attack during the same monitoring period.

The pain free period after applying N2 ranged from one hour to seventy two hours (the full three days of self-monitoring) with a mean of 26.02$\pm$16.26 hours. The longest pain free duration was experienced by Post Operative pain cases (52$\pm$14.32 hours), followed by shingles cases (46.02$\pm$24.34 hours). The least pain free duration was recorded by Pulled ligaments cases (2.87$\pm$1.56 hours). The difference in the pain free period duration between the seven groups was statistically significant P=.0001

70 patients using N2 entered their sleep pattern on questionnaire. 68.5% (n=48) slept through the night. 24.5% (n=17) experienced slight sleep disturbance and 7.0% (n=5) experienced severe disturbance.

68 patients entered their analgesic drug preference in the questionnaire. 75% (n=51) stated N2 Is more effective than conventional drugs they used in the past due to its instant action and ease of use, 10.3% (n=7) stated that the conventional pain killers are more effective. 14.7 % (n=10) found no difference between the two. No adverse events were recorded.

Example 3:

The aerosol dispenser contained the following:

| INGREDIENTS | % BY WEIGHT |
|---|---|
| Water | 59 % |
| Methylal | 6.2% |
| Ethanol | 5.1% |
| Menthol | 1.5% |
| Indomethacin | 0.2% |
| Dimethyl Ether | 28 % |
| Pressure at 20° C : 4.1 bar Particle size : 30 microns Discharge Rate at 20°C : 0.8 gm/sec Discharge angle at 20° C : 33° pH: 7.8 Viscosity at 20°C: 0.82 | 100% |

Example 4 :

The aerosol dispenser contained the =following:

| INGREDIENTS | % BY WEIGHT |
|---|---|
| Water | 62.85% |
| Methylal | 5.1% |
| Ethanol | 5.0% |
| Menthol | 1.5% |
| Methyl salicylate | 0.2% |
| Sodium Benzoate/ Disodium dodecenyl-sulfo-succinate | 0.35% |
| Dimethyl Ether | 25% |
| Pressure at 20° C : 4.5 bar Particle size : 60 microns Discharge Rate at 20°C : 0.85 gm/sec Discharge angle at 20° C : 31° pH: 8.2 Viscosity at 20°C: 0.77 | 100% |

Tests were carried out to compare the result of each stimulus i.e. cooling, mechanical and chemical acting independently and consecutlvely, using products available on the market e.g. skin cooling products, TENS or home therapy as detailed above, and using the aerosol apparatus as described In Example one or Example two where the three stimuli (cooling, mechanical and chemical) or two stimuli (cooling and mechanical) respectively act simultaneously on the same area of the same patients. The simultaneous application of the three or two stimuli yielded dramatic results and proved to be far more effective than the three or two independent stimuli acting independently and consecutively. Patient compliance, including children, when applying the simultaneous stimuli proved to be superior due to the convenience, ease of use and portability. The apparatus of the embodiment of the invention detailed in examples one and two is non-steroidal and the particles do not stain the fabric. No adverse events were recorded In the three clinical studies.

Not only did the aerosol apparatus of the present invention ease the symptoms but, as proven in the clinical study two above, it treated a chronic disease e.g. atopic dermatitis which none of the above stimuli acting independently are known to treat. The results of the embodiment aerosol apparatus containing the composition of the present invention in Clinical Study two were significant and superior to the results of the traditional treatment Hydrocortisone 1%. Moreover throughout the Clinical Studies, one, two and three detailed above, there were no side effects and the patient compliance of the apparatus of the present invention was superior to the established traditional treatment e.g. steroldal hydrocortisone 1% or known cooling products or home therapies.

The aerosol apparatus and composition according to the present invention can be effective in treating any one or more of the following diseases and symptoms of diseases. The invention Is not however to be limited thereto ;

Chronic or acute pain related to rheumatic and muscular pain, arthritis, rheumatoid arthritis, osteoarthritis, bell's palsy, upper or lower back pain, lumbago, fibromyalgia, cluster, tension or other headaches, migraines, menstrual pain (dysmenorrhea), neck pain, sciatica, shingles (PHN), labor pain, trigeminal neuralgia, whiplash, neck injury, toothache, sports injuries, Peripheral Nerve Injuries, aching joints, Phantom Limb Pain, post operative pain, muscular pain, burning, stinging or throbbing pain. Systemic Pain e.g. Bursitis, Causalgia, Multiple Sclerosis, Fibrositis, Neuralgia, Raynaud's Syndrome, Synovitis, Diabetic Peripheral Neuropathy. Head and Neck pain e.g. Dental Disorders, Spondylosis, Sprains/ Strains, Subocclpltal Headaches, TMJ Syndrome, Torticollis. Abdominal Pain: Diverticulosis. Back Pain:Facet Syndrome, IVD Syndrome, Lumbosacral Pain, Radiculitis, Thoracodynia. Lower Extremity Pain: Ankle Pain, Foot Pain, Fractures, Ischialgia, Knee Pain, Passive Stretch Pain, Tendonitis, Thrombophlebitis, pulled ligaments, gout, sore feet. Upper Extremity Pain: Epicondylitis, Frozen Shoulder, Hand Pain, Wrist Pain.

Prurltls, general or localized, skin dryness, lichenification, cracking, erythema, exudation, excoriation, wheals, scales, ulcers, papules, vesicles, edema, crusts, scabies, urticaria, eczema, atopic dermatitis and neurodermatitis, lichen planus, dermatitis herpetiformis, psoriasis, pityriasis rosea, xerosis, personal itching, itching related to systemic disorders e.g. drug reaction, allergy, cholestasis. Bullous pemphigoid, bacterial and viral infedions, herpes, sunburn, insect bites, sumac dermatitis. Contact dermatitis, prickly heat, hand eczema, skin rash, skin irritation, skin eruption, cutaneous flushing, chicken pox, shingles, fungus, infections, skin conditions related to pregnancy and nursing, menopausal symptoms.

The above results of the clinical studies related to the invention are directed primarily to preferred embodiments and pradices thereof. It will be readily apparent to those skilled in the art that further changes and modifications in the actual implementation of the concepts described herein can readlly be made without departing from the spirit and scope of the invention as defined by the following claims.

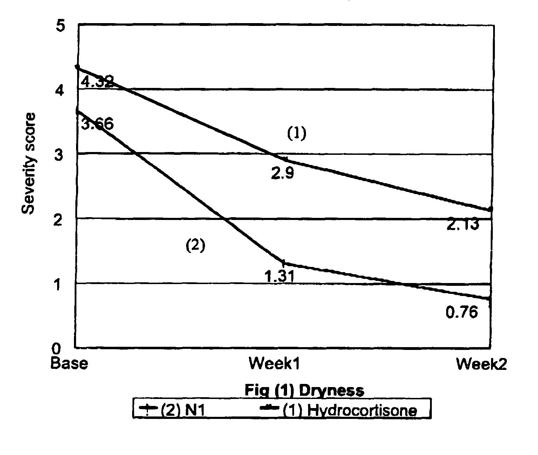

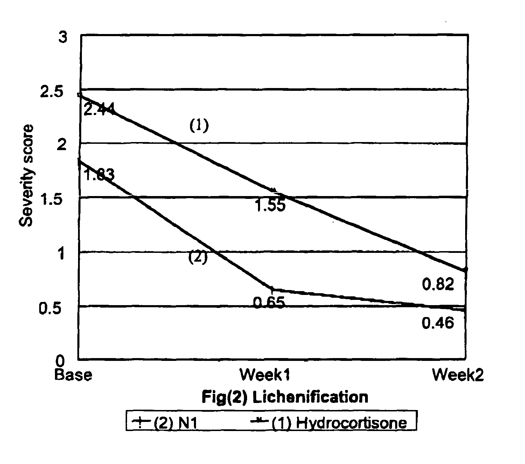

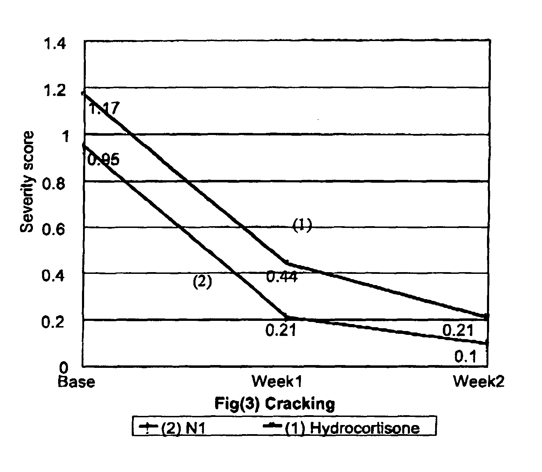

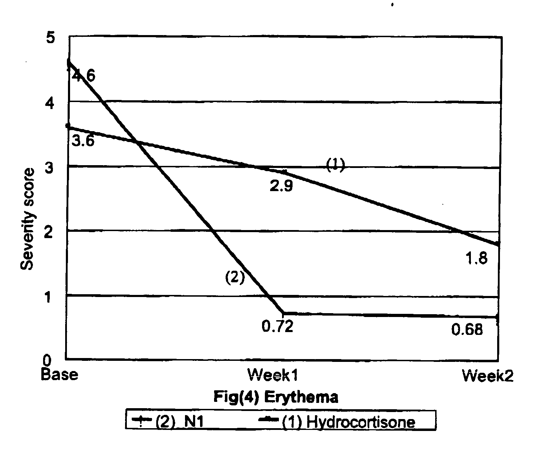

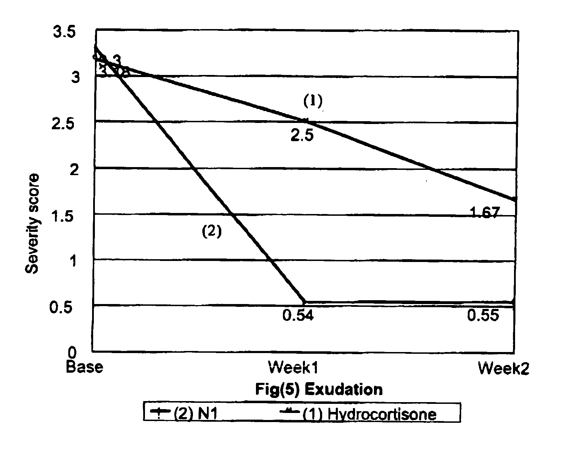

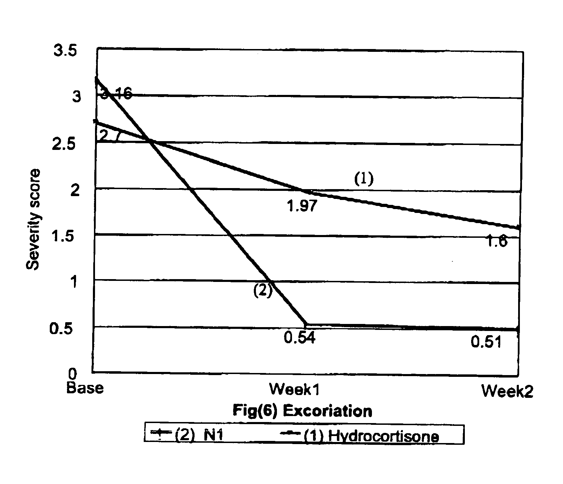

## Claims

1.  An aerosol apparatus containing a non-flammable, water-based composition, the viscosity of which composition is from 0.2 to 30 mPa.s at 20° C, which apparatus is adapted in use to release cool water-based particles for the simultaneous topical application of thermal and mechanical kinetic energy stimuli for skin or body tissue cooling and for the generation of pulses In the body respectively, at a pressure of from 0.5 to 11 bars at 20° C which pressure Is created by using dimethyl ether or liquefied petroleum gas or a mixture of the two to discharge said water-based particles at a rate of from 0.1 to 8 gm/sec at 20° C at a discharge angle of from 0.0° to 145° at 20° C, at a temperature which is higher than 0°C and lower than the ambient temperature and with a particle diameter of from 10 to 600 microns.

2.  An apparatus according to claim 1 which includes pressurized gas.

3.  An apparatus according to Claim 1 or claim 2, wherein the cool water-based particles which the apparatus of the present invention is adapted In use to produce have a pH of from 7.01 - 12.6, thereby also to apply chemical stimuli on the skin or body tissues.

4.  An apparatus according to any one of the preceding claims, which apparatus is adapted in use to release cool water-based particles at a pressure of from 1.0 to 8.0 bar at 20° C.

5.  An apparatus according to any one of the preceding claims, which apparatus is adapted in use to release cool water-based particles with a diameter of from 15 to 300 microns.

6.  An apparatus according to any one of the preceding claims, which apparatus is adapted in use to release cool water-based particles with a diameter of from 10 to 250 microns.

7.  An apparatus according to any one of the preceding claims, which apparatus Is adapted in use to release cool water-based particles at a discharge rate of from 0.4 to 3 gm/sec at 20° C.

8. An apparatus according to any one of the preceding claims, which apparatus is adapted in use to release cool water-based particles at a discharge rate of from 0.2 to 5 gm/sec at 20° C.

9. An apparatus according to any one of the preceding claims, which apparatus is adapted in use to release cool water-based particles at a discharge angle of from 5 to 60° at 20° C.

10. An apparatus according to any one of the preceding claims, the viscosity of the water-based composition which it contains being from 0.3 to 15 mPa.s. at 20° C.

**Patentansprüche**

1. Aerosolvorrichtung, die eine nicht entflammbare Zusammensetzung auf Wasserbasis enthält, wobei die Viskosität dieser Zusammensetzung im Bereich von 0,2 bis 30 mPa.s bei 20°C liegt, wobei die Vorrichtung die Aufgabe hat, beim Betrieb kühle Partikel auf Wasserbasis für die gleichzeitige topische Applikation thermischer und mechanischer Reize von kinetischer Energie zum Kühlen von Haut- und Körpergewebe oder zur Erzeugung von Impulsen im Körper mit einem Druck von 0,5 bis 11 Bar bei 20°C freizusetzen, wobei der Druck mittels Dimethylether oder verflüssigtem Erdölgas oder einer Mischung aus den beiden erzeugt wird, um die genannten Partikel auf Wasserbasis mit einer Geschwindigkeit im Bereich von 0,1 bis 8 g/sec bei 20°C in einem Abgabewinkel im Bereich von 0,0° bis 145° bei 20°C bei einer Temperatur, die über 0°C und unter der Umgebungstemperatur liegt, und mit einem Partikeldurchmesser im Bereich von 10 bis 600 $\mu$m abzugeben.

2. Vorrichtung nach Anspruch 1, die Druckgas enthält.

3. Vorrichtung nach Anspruch 1 oder Anspruch 2, wobei die kühlen Partikel auf Wasserbasis, die die erfindungsgemäße Vorrichtung beim Betrieb erzeugen soll, einen pH-Wert im Bereich von 7,01 bis 12,6 haben und **dadurch** auch chemische Reize auf Haut- oder Körpergewebe applizieren.

4. Vorrichtung nach einem der vorherigen Ansprüche, wobei die Vorrichtung die Aufgabe hat, beim Betrieb kühle Partikel auf Wasserbasis mit einem Druck im Bereich von 1,0 bis 8,0 Bar bei 20°C freizusetzen.

5. Vorrichtung nach einem der vorherigen Ansprüche, wobei die Vorrichtung die Aufgabe hat, beim Betrieb kühle Partikel auf Wasserbasis mit einem Durchmesser im Bereich von 15 bis 300 $\mu$m freizusetzen.

6. Vorrichtung nach einem der vorherigen Ansprüche, wobei die Vorrichtung die Aufgabe hat, beim Betrieb kühle Partikel auf Wasserbasis mit einem Durchmesser im Bereich von 10 bis 250 $\mu$m freizusetzen.

7. Vorrichtung nach einem der vorherigen Ansprüche, wobei die Vorrichtung die Aufgabe hat, beim Betrieb kühle Partikel auf Wasserbasis mit einer Abgabegeschwindigkeit im Bereich von 0,4 bis 3 g/sec bei 20°C freizusetzen.

8. Vorrichtung nach einem der vorherigen Ansprüche, wobei die Vorrichtung die Aufgabe hat, beim Betrieb kühle Partikel auf Wasserbasis mit einer Abgabegeschwindigkeit im Bereich von 0,2 bis 5 g/sec bei 20°C freizusetzen.

9. Vorrichtung nach einem der vorherigen Ansprüche, wobei die Vorrichtung die Aufgabe hat, beim Betrieb kühle Partikel auf Wasserbasis in einem Abgabewinkel im Bereich von 5 bis 60° bei 20°C freizusetzen.

10. Vorrichtung nach einem der vorherigen Ansprüche, wobei die Viskosität der auf Wasser basierenden Zusammensetzung, die sie enthält, im Bereich von 0,3 bis 15 mPa.s. bei 20°C liegt.

**Revendications**

1. Un appareil aérosol contenant un composé aqueux non inflammable d'une viscosité de 0,2 à 30 mPa.s à 20 °C, ledit appareil étant adapté pour délivrer, en cours d'utilisation, des particules aqueuses froides pour l'application topique simultanée de stimuli d'énergie cinétique thermique et mécanique destinés à refroidir les tissus cutanés ou corporels et générer des impulsions dans le corps à une pression de 0,5 à 11 bars à 20 °C, respectivement, ladite pression étant créée par l'utilisation d'oxyde de méthyle ou de gaz de pétrole liquéfié ou d'un mélange des deux pour délivrer lesdites particules aqueuses à un débit de 0,1 à 8 gm/s. à 20 °C à un angle de décharge de 0,0° à 145° à 20 °C, à une température supérieure à 0 °C et inférieure à la température ambiante, avec des particules d'un diamètre de 10 à 600 micromètres.

2. Un appareil selon la Revendication 1 qui inclut le gaz sous pression.

3. Un appareil selon la Revendication 1 ou la Revendication 2, dans lequel le pH des particules aqueuses froides que l'appareil de la présente invention est adapté à produire en cours d'utilisation, est de 7,01 à 12,6, ledit appareil étant donc également adapté à appliquer des stimuli sur les tissus cutanés et corporels.

4. Un appareil selon l'une des revendications précédentes, ledit appareil étant adapté à délivrer, en cours d'utilisation, des particules aqueuses formées à froides à une pression de 1,0 à 8,0 bars à 20 °C.

5. Un appareil selon l'une des revendications précédentes, ledit appareil étant adapté à délivrer, en cours d'utilisation, des particules aqueuses froides d'un diamètre de 15 à 300 micromètres.

6. Un appareil selon l'une des revendications précédentes, ledit appareil étant adapté à délivrer, en cours d'utilisation, des particules aqueuses froides d'un diamètre de 10 à 250 micromètres.

7. Un appareil selon l'une des revendications précédentes, ledit appareil étant adapté à délivrer, en cours d'utilisation, des particules aqueuses froides à un débit de décharge de 0,4 à 3 gm/s. à 20 °C.

8. Un appareil selon l'une des revendications précédentes, ledit appareil étant adapté à délivrer, en cours d'utilisation, des particules aqueuses froides à un débit de décharge de 0,2 à 5 gm/s. jusqu'à 20 °C.

9. Un appareil selon l'une des revendications précédentes, ledit appareil étant adapté à délivrer, en cours d'utilisation, des particules aqueuses froides à un angle de décharge de 5° à 60° à 20 °C.

10. Un appareil selon l'une des revendications précédentes, dont la viscosité de la composition aqueuse qu'il contient est de 0,3 à 15 mPa.s à 20 °C.

**Fig (1) Dryness**

─┼─ (2) N1      ─▲─ (1) Hydrocortisone

Fig(2) Lichenification

+ (2) N1    ☀ (1) Hydrocortisone

Fig(3) Cracking

(2) N1    (1) Hydrocortisone

Fig(4) Erythema

─┼─ (2) N1          ─ (1) Hydrocortisone

Fig(5) Exudation

(2) N1    (1) Hydrocortisone

Fig(6) Excoriation

(2) N1    (1) Hydrocortisone

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1195173 A, Daizo Corporation

### Non-patent literature cited in the description

- **FRUHSTORFER H ; HERMANNS M ; LATZKE L.** Effects of Thermal Stimulation on Clinical and Experimental Itch. *Pain,* February 1986, vol. 24 (2), 259-69
- **FRUHSTORFER H ; HERMANNS M ; LATZKE L.** Effects of Thermal Stimulation on Clinical and Experimental itch. *Pain,* February 1986, vol. 24 (2), 259-69
- **BROMM B. ; SCHAREIN E. ; DARSOW U. ; RING J.** Effects of menthol and cold on histamine-induced itch and skin reactions In man. *Neurosci. Lett,* 1995, vol. 187, 157-160
- **MADDEN EJ ; ITCH, J.** *Pain Symptom Management,* 1986, vol. 1 (2), 97-9
- **LITT JZ.** Topical treatments of itching without corticosteroids. *Itch Mechanisms and management of Pruritis,* 1994, 383-397
- **EKBLOM A ; FJELLNER B ; HANSSON P.** The influence of mechanical vibratory stimulation and transcutaneous electromechanical nerve stimulation on experimental pruritus induced by histamine. *Acta Physiol Scand,* 1984, vol. 122, 361-7
- **EKBLOM A ; FJELLNER B ; HANSSON P.** The influence of extrasegmental mechanical vibratory stimulation and transcutaneous electromechanical nerve stimulation on experimental pruritus induced by histamine. *Acta Physiol Scand,* 1984, vol. 122, 361-7
- **JONES JB.** Six area, six sign atopic dermatitis (SASSAD) severity score : A simple system for monitoring disease activity in atopic dermatitis. *Br J Dermatol.,* 1996, vol. 135, 25-30
- **HANIFIN J.M. ; RAJKA G.** Diagnostic Features of Atopic Dermatitis. *Acta Derma (Stock,* 1980, 44-47